# EUROPEAN PATENT SPECIFICATION

(11) **EP 2 784 631 B1**
(45) Date of publication and mention of the grant of the patent: **18.05.2016**
(21) Application number: 14160396.9
(22) Date of filing: 18.03.2014
(51) Int. Cl.: A61B 5/0488, A61B 5/11, G06F 3/01, A61B 5/00, G01B 21/16, G01P 13/00

(54) **Method and wearable device to sense motion of user**
Verfahren und tragbare Vorrichtung zur Erfassung der Bewegung eines Benutzers
Procédé et dispositif portable pour détecter un mouvement d'utilisateur

(30) Priority: 25.03.2013 KR 20130031422
(43) Date of publication of application: 01.10.2014
(73) Proprietor: Samsung Electronics Co., Ltd., Suwon-si Gyeonggi-do 443-742 (KR)
(72) Inventor: KIM, Sang Joon, Gyeonggi-do 446-712 (KR); MUN, Min Young, Gyeonggi-do 446-712 (KR); CHOI, Chang Mok, Gyeonggi-do 446-712 (KR)
(74) Representative: Grünecker Patent- und Rechtsanwälte PartG mbB

(56) References cited:
- FR-A1- 2 965 060
- US-A1- 2008 223 131
- US-A1- 2008 300 055
- Ami LUTTWAK: "Human Motion Tracking and Orientation Estimation using inertial sensors and RSSI measurements", , 28 December 2011 (2011-12-28), XP055133501, Retrieved from the Internet: URL:http://www.cs.huji.ac.il/~dolev/pubs/t hesis/ami-msc-thesis.pdf [retrieved on 2014-08-06]
- HAMIE J ET AL: "Constrained decentralized algorithm for the relative localization of wearable wireless sensor nodes", 2013 IEEE SENSORS, IEEE, 28 October 2012 (2012-10-28), pages 1-4, XP032308702, ISSN: 1930-0395, DOI: 10.1109/ICSENS.2012.6411246
- STUPP G ET AL: "The expected uncertainty of range-free localization protocols in sensor networks", THEORETICAL COMPUTER SCIENCE, AMSTERDAM, NL, vol. 344, no. 1, 11 November 2005 (2005-11-11), pages 86-99, XP027654934, ISSN: 0304-3975 [retrieved on 2005-11-11]

## Description

### BACKGROUND

### 1. Field

The following description relates to a method and a wearable device to sense a motion of a user through relative position estimation and motion sensing of a wearable device operatively attached to clothes or a body of the user.

### 2. Description of Related Art

Currently, portable electronic devices, such as smart phones, tablet personal computers (PCs), and personal digital assistants (PDAs), for example, are increasingly used. A technology to interpret a body motion of a user is desired to enhance portability of the electronic device, and to conveniently control the electronic device.

A method to interpret the body motion of the user may include attaching a motion sensing acceleration sensor to the electronic device. Such method, however, is suitable to interpret a simple motion rather than a complex motion of the user.

Another method to interpret image information may be performed by attaching a motion sensing camera to the electronic device. In this case, because the user needs to be present in front of the device, the user needs to separately manage the electronic device from a body of the user.

Document A. Luttwak: "Human motion tracking and orientation estimation using inertial sensors and RSSI measurements", Thesis, School of Engineering and Computer Science, The Hebrew University of Jerusalem, Israel, 28 December 2011, from the Internet, URL: http//www.cs.huji.ac.il/~dolev/pubs/thesis/ami-msc-thesis.pdf, mentions a system which consists of a single mobile node and N static nodes, referred to as anchor nodes. The anchor nodes, located in the transmission range of the mobile node, calculate the received power values (RSSI). The mobile node is also equipped with inertial sensors which record the node's inertial data. The goal is to continuously estimate the mobile node's location from the mobile node inertial data and from the attenuation of the electromagnetic signal in space.

Document J. Hamie et al.:"Constraint decentralized algorithm for the relative localization of wearable wireless sensor nodes", 2013 IEEE Sensors, IEEE, 28 October 2012, pages 1 - 4, ISSN 1930-0395, DOI 10.1109/ICSENS.2012.6411246 teaches wireless body sensor networks where wireless devices placed on the body can be classified into two categories. Simple mobile nodes with unknown positions must be located relatively to reference anchor nodes, which are attached onto the body at known and reproducible positions.

Document US 2008/0300055 A1 deals with a wristband which a player may have. The wristband may include motion sensors, such as accelerometers, for detecting motions. The wristband may interpret motions as commands for a device with which the wristband is in communication.

Document FR 2 965 060 A1 deals with a method of localizing emitters or receivers which are attached to a mobile body. The method is characterized by comprising a step of measuring the distances separating the first nodes attached to a first body from second nodes by using wireless means, wherein a second node may be a second mobile body which is remote but within the transmission range of the first mobile body.

US 2008/0223131 A1 provides a system and method for capturing motion of an object, e.g., a person. A set of ultrasonic signal sources and signal sensors are arranged on a user. The system continuously measures pairwise distances between the sources and the sensors.

The document: G. Stupp, M. Sidi: "The expexted uncertainty of range-free localization protocols in sensor networks", Theoretical Computer Science, Amsterdam, NL, vol. 344, no. 1, 11 November 2005, pages 86 - 99, ISSN 0304-3975, considers three range-free localization protocols for sensor networks and analyzes their accuracy in terms of the expected area of uncertainty of position per sensor.

### SUMMARY

This Summary is provided to introduce a selection of concepts in a simplified form that are further described below in the Detailed Description. This Summary is not intended to identify key features or essential features of the claimed subject matter, nor is it intended to be used as an aid in determining the scope of the claimed subject matter.

In accordance with an illustrative configuration, there is provided a wearable device, including a motion sensor configured to sense a motion signal from a motion of a user of the wearable device; and a processor configured to calculate a motion of the wearable device based on the motion signal and position related information received from a neighbor wearable device; and a magnetic near field communicator configured to receive the position related information from the neighbor wearable device and transmit the position related information to the processor, wherein the position related information comprises at least one of a distance from the wearable device estimated by the neighbor wearable device, a motion signal of the neighbor wearable device, and a position of the neighbor wearable device.

The processor may be further configured to estimate a position of the wearable device based on the motion signal.

The processor may be further configured to correct the estimated position of the wearable device based on the position related information so that an error range of the estimated position is less than a threshold.

The error range may be an overlapping space of positions of the wearable device estimated based on distances from at least two neighbor wearable devices calculated from the position related information.

The processor may be further configured to estimate a distance from the neighbor wearable device based on a power level of a reference signal included in the position related information, and to calculate a position of the wearable device based on the estimated distance and the position related information.

The processor may be further configured to sense the motion of the user by tracking a position of the wearable device, in time series, based on the motion signal and the position related information.

The position related information may include at least one of a distance from the wearable device estimated by the neighbor wearable device, a motion signal of the neighbor wearable device, and a position of the neighbor wearable device.

The motion sensor may include at least one of an acceleration sensor and an electromyogram (EMG) sensor.

The wearable device may further include a body applier configured to apply the wearable device to a body of the user.

The wearable device may further include a magnetic near field communicator configured to receive the position related information from the neighbor wearable device and transmit the position related information to the processor.

In accordance with a further configuration, there is provided a method of a wearable device, the method including sensing a motion signal from a motion of a user of the wearable device; and calculating a motion of the wearable device based on the motion signal and position related information from a neighbor wearable device.

The sensing may include estimating a position of the wearable device based on the motion signal.

The calculating may include correcting the estimated position of the wearable device based on the position related information so that an error range of the estimated position is less than a threshold.

The correcting may include detecting a power level of a reference signal included in the position related information; estimating a distance of the wearable device from the neighbor wearable device based on the detected power level; and determining the error range based on the motion signal and the distance from the neighbor wearable device.

The calculating may include estimating a distance of the wearable device from the neighbor wearable device by analyzing a power level of a reference signal included in the position related information; and calculating a position of the wearable device based on the estimated distance from the neighbor wearable device and a position of the neighbor wearable device that is included in the position related information.

In accordance with an illustrative configuration, there is provided a method of a wearable device, the method including estimating a position of the wearable device based on a motion signal sensed according to a motion of a user; and calculating a motion of the wearable device based on the estimated position of the wearable device and position related information from at least two neighbor wearable devices.

The calculating may include estimating distances from the at least two neighbor wearable devices based on the position related information; and correcting the estimated position of the wearable device by determining, as an error range, an overlapping space of positions of the wearable device based on the distances from the at least two neighbor wearable devices.

The calculating may include determining the corrected position of the wearable device as a finally estimated position of the wearable device when the error range is less than a threshold.

In accordance with an illustrative configuration, there is provided a method of a wearable device, including estimating a position of the wearable device based on a motion signal of the wearable device; calculating a motion of the wearable device based on the estimated position and position related information from at least two neighbor wearable devices; and correcting the estimated position of the wearable device to have an error range less than a threshold.

The error range may be an overlapping space of positions of the wearable device 200 estimated based on distances from the at least two neighbor wearable devices calculated from the position related information.

The method may further include estimating a distance from the neighbor wearable device based on a power level of a reference signal included in the position related information, and calculating a position of the wearable device based on the estimated distance and the position related information.

The method may further include updating the position of the wearable device as a finally estimated position of the wearable device in response to the error range being less than the threshold.

The estimating of the position of the wearable device may include calculating a distance and a direction travelled from a previous position to a current position of the wearable device based on a double integration of an acceleration signal.

In accordance with an illustrative configuration, there is provided a non-transitory computer-readable medium including at least one program for instructing a computer to perform the method as described above.

Other features and aspects will be apparent from the following detailed description, the drawings, and the claims.

### BRIEF DESCRIPTION OF THE DRAWINGS

FIG. 1 is a diagram illustrating an example of wearable device sensing a motion of a user, in accordance with an embodiment.
FIG. 2 is a block diagram illustrating an example of the wearable device sensing the motion of the user, in accordance with an embodiment.
FIG. 3 is a view to describe another example of a wearable device sensing the motion of the user, in accordance with an alternative embodiment.
FIG. 4 is a view to describe another example of a wearable device sensing the motion of the user, in accordance with a further alternative embodiment.
FIG. 5 is a flowchart illustrating an example of a method sensing a motion of the user of a wearable device, in accordance with an embodiment.
FIG. 6 is a flowchart illustrating another example of a method sensing a motion of the user of a wearable device, in accordance with an alternative embodiment.
FIG. 7 is a diagram to describe an example of a process of a wearable device to estimate a position, in accordance with an embodiment.

Throughout the drawings and the detailed description, unless otherwise described, the same drawing reference numerals will be understood to refer to the same elements, features, and structures. The relative size and depiction of these elements may be exaggerated for clarity, illustration, and convenience.

### DETAILED DESCRIPTION

The following detailed description is provided to assist the reader in gaining a comprehensive understanding of the methods, apparatuses, and/or systems described herein. Accordingly, various changes, modifications, and equivalents of the methods, apparatuses, and/or systems described herein will be suggested to those of ordinary skill in the art. The progression of processing steps and/or operations described is an example: however, the sequence of and/or operations is not limited to that set fourth herein and may be changed as is known in the art, with the exception of steps and/or operations necessarily occurring in a certain order. Also, description of well-known functions and constructions may be omitted for increased clarity and conciseness.

Sensing a motion of a user may include using an acceleration sensor or an electromyogram (EMG) sensor. However, such sensors may lack accuracy and may sense only ON/OFF of a motion, and also relatively large in size or volume. In ON/OFF of the motion, ON may indicate a case in which the motion occurs and OFF may indicate a case in which the motion does not occur.

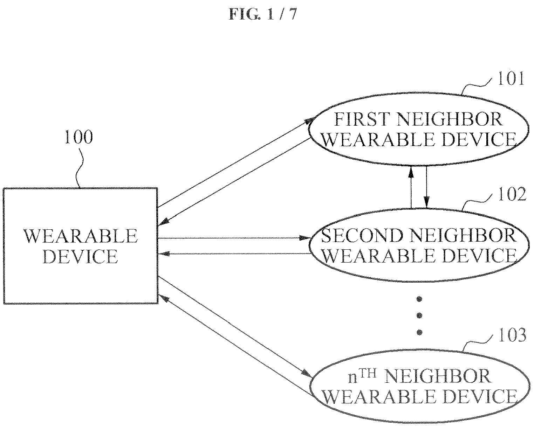

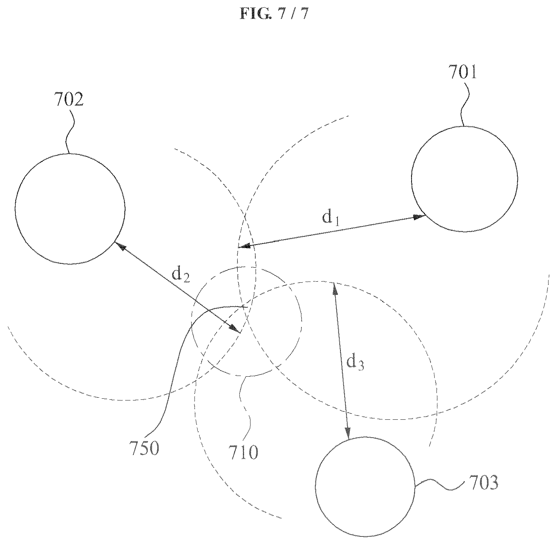

FIG. 1 illustrates an example of a wearable device 100 to sense a motion of a user. The motion of the user may include a change in position of the wearable device 100 that occurs in response to a motion of the user. The term "position" used herein may indicate a position range/coverage and may be expressed as an approximate range as illustrated in FIG. 7, to be later described.

The wearable device 100, according to an embodiment, may decrease an error range by correcting a position of the wearable device 100 estimated from a movement or a motion of the user. The wearable device 100 would correct the position estimated from position related information received from neighbor wearable devices, for example, a first neighbor wearable device 101, a second neighbor wearable device 102, and an n^{th} neighbor wearable device 103. The motion of the user may be precisely sensed by further estimating a position of the wearable device 100. For example, using a plurality of subminiature devices in the wearable device 100, it is possible to enhance use convenience while precisely sensing a motion according of the user.

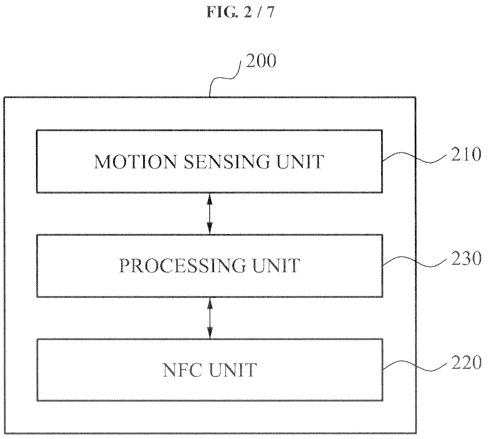

The wearable device 100 initially estimate its position or the position of the wearable device 100 by sensing a gesture or the motion of the user using a motion sensing unit (FIG. 2) embedded within the wearable device 100. The wearable device 100 may finally correct the estimated position based on position related information received from the neighbor wearable devices, for example, the first neighbor wearable device 101, the second neighbor wearable device 102, and the n^{th} neighbor wearable device 103. In one example, the corrected position of the wearable device 100 may be the finally estimated position of the wearable device 100.

The wearable device 100 may receive position related information of a position corresponding to another wearable device from each of the first neighbor wearable device 101, the second wearable device 102, and the n^{th} wearable device 103. In this example, the first neighbor wearable device 101, the second neighbor wearable device 102, and the n^{th} neighbor wearable device 103 may be neighbor wearable devices that are positioned to be adjacent to the wearable device 100. Also, "n" may denote a natural number of greater than or equal to "1". The number of neighbor wearable devices may vary depending on a degree of accuracy needed, a particular body part of the user being analyzed for estimated position, and a cost associated with an implementation of the neighbor wearable devices.

In an example, position related information may include a distance from the wearable device 100 to a neighbor wearable device, a motion signal of the neighbor wearable device, a position of the neighbor wearable device, and additional information associated with the position of the neighbor wearable device. In this example, the distance from the wearable device 100 to the neighbor wearable device may include a distance estimated by analyzing a power level of a reference signal received at the neighbor wearable device from the wearable device 100.

Similar to the wearable device 100, the neighbor wearable device may also autonomously correct motion sensing by receiving position related information from the wearable device 100 and other neighbor wearable devices. For example, the first neighbor wearable device 101 may receive position related information from the wearable device 100, the second neighbor wearable device 102, and the n^{th} neighbor wearable device 103.

An example of a wearable device to estimate and correct a position of the wearable device by sensing a motion of a user will be described.

FIG. 2 illustrates an example of a wearable device 200 sensing the motion of the user, in accordance with an embodiment.

Referring to FIG. 2, the wearable device 200 includes a motion sensing unit or a motion sensor 210, a magnetic near field communication (NFC) unit or near field communicator (NFC) 220, and a processing unit or processor 230.

The motion sensing unit 210 senses a motion signal in relation to a motion of a user. The motion signal may include at least one of an acceleration signal, an EMG signal, and a signal that occurs in relation to the motion of the user. For example, the motion sensing unit 210 may include an acceleration sensor, an EMG sensor, or a sensor enabled to sense the motion of the user and produce the motion signal indicative thereof.

The NFC unit 220 receives position related information from a neighbor wearable device. The NFC unit 220 communicates with the neighbor wearable device using an NFC channel. For example, the NFC unit 220 receives position related information or transmits a request to the neighbor wearable device to transmit position related information, where the processing unit 230 controls the NFC unit 220 to transmit the request.

The processing unit 230 calculates a motion of the wearable device 200 based on the motion signal and the position related information. The processing unit 230 estimates a position of the wearable device 200 based on the motion signal. The processing unit 230 determines a finally estimated position of the wearable device 200 by correcting the estimated position of the wearable device 200 so that an error range of the estimated position is less than a threshold. For example, the threshold refers to a value that guarantees a level of accuracy capable of interpreting a predetermined gesture or a predetermined motion defined to control an electronic device, such as the wearable device 200. The error range may be an overlapping space of positions of the wearable device 200 estimated based on distances from at least two neighbor wearable devices calculated from the position related information. The overlapping space of positions of the wearable device 200 estimated based on the distances will be described with reference to FIG. 7.

The processing unit 230 estimates a distance from the neighbor wearable device based on a power level of a reference signal included in the position related information, and calculates a position of the wearable device 200 based on the estimated distance and the position related information.

The processing unit 230 determines the motion of the user by tracking a position of the wearable device 200, in time series, based on the motion signal and the position related information.

The wearable device 200 may further include a body applier (not shown) configured to enable the wearable device 200 to be mounted, attached, or applied to a body of the user. For example, the body applier may include a body attachment unit, such as a module, configured to attach the wearable device 200 to a fingernail of the user, and a body fixing unit, such as a module, configured to fix the wearable device 200 to a finger, a face, a waist, and a forearm.

The wearable device 200 may include a non-transitory recording medium including at least one program for instructing a computer to perform a method to sense a motion of a user.

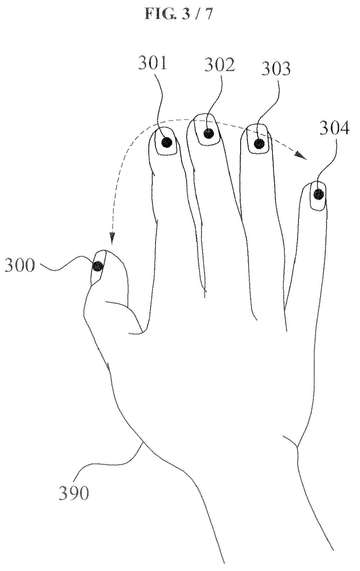

FIG. 3 illustrates another example of a wearable device 300 sensing the motion of the user, in accord with an embodiment.

Referring to FIG. 3, the wearable device 300 and neighbor wearable devices 301, 302, 303. and 304 are attached to fingernails of a user 390.

The wearable device 300 and each of the neighbor wearable devices 301, 302, 303, and 304 correct a corresponding position, which is estimated through self-motion sensing, by mutually exchanging position related information. For example, a motion sensing unit (such as the motion sensing unit 210 of FIG. 2) in the wearable device 300 analyzes a motion of an individual finger, and estimates a distance between the wearable device 300 and each of the neighbor wearable devices 301, 302, 303, and 304 based on signal attenuation occurring due to a physical characteristic of magnetic near field communication. In this example, a distance from each of the neighbor wearable devices 301, 302, 303, and 304 may be estimated with a resolution of centimeter (cm) or millimeter (mm) level based on a detected power level of a reference signal included in the position related information.

For example, the wearable device 300 attached to a thumbnail may estimate a position of the wearable device 300 based on distances from relative positions of the neighbor wearable devices 301, 302, 303, and 304 attached to individual fingers, respectively. By tracking the estimated position of each of the wearable device 300 and the neighbor wearable devices 301, 302, 303, and 304 in time series, it is possible to collectively sense motions of fingers, a hand, and an arm.

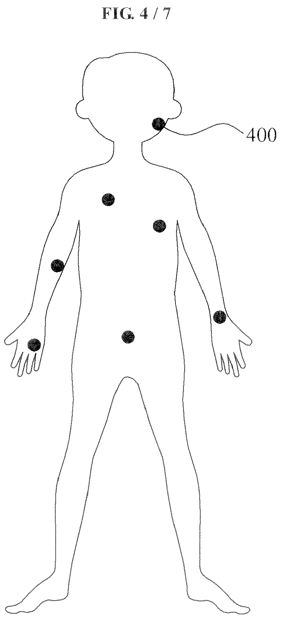

FIG. 4 illustrates another example of a wearable device 400 sensing the motion of the user, in accordance with a further alternative embodiment.

Referring to FIG. 4, the wearable device 400 is attached and mounted to various portions of a body of a user, and is attached and mounted without constraints on a predetermined shape.

The wearable device 400 includes accessories applied to the body or clothes of the user. For example, the accessories may include a ring, a wrist watch, glasses, a bracelet, an ankle bracelet, a necklace, an earring, and other accessory styles. A device applied to clothes of the user may include a clothing-type wearable computer.

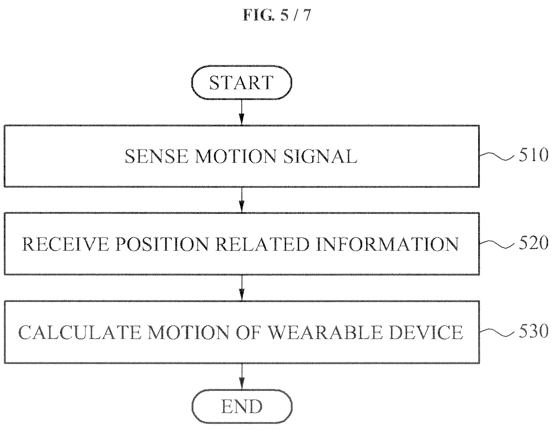

FIG. 5 illustrates an example of a method sensing the motion of the user of a wearable device, in accordance with an embodiment.

In operation 510, the method senses a motion signal according to a motion of the user through a motion sensing unit. The method estimates a position of the wearable device based on the motion signal using a processing unit.

In operation 520, the method of the wearable device receives position related information from a neighbor wearable device using a magnetic near field communication (NFC) unit. For example, the position related information may include information associated with a distance from the wearable device estimated by the neighbor wearable device, a motion signal of the neighbor wearable device, and a position of the neighbor wearable device. The position of the neighbor wearable device may include a position that is estimated based on a motion signal sensed using a motion sensing unit in the neighbor wearable device.

In operation 530, the method calculates a motion of the wearable device based on the motion signal and the position related information using the processing unit. The method corrects the estimated position of the wearable device based on the position related information, so that an error range of the estimated position is less than a threshold. For example, the method detects a power level of a reference signal in the position related information, estimates a distance of the wearable device from the neighbor wearable device based on the detected power level of the reference signal, and determines the error range based on the motion signal and the distance from the neighbor wearable device. The method estimates the distance from the neighbor wearable device based on the power level of the reference signal using a signal attenuation characteristic of an NFC channel.

To calculate the motion of the wearable device, the method estimates the distance from the neighbor wearable device by analyzing the power level of the reference signal included in the position related information using the processing unit. The method calculates the position of the wearable device based on the distance from the wearable device to the neighbor wearable device and a position of the neighbor wearable device in the position related information.

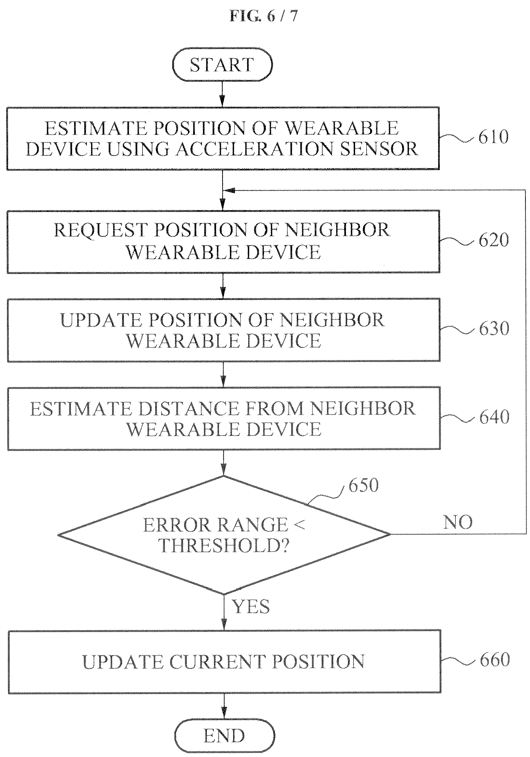

FIG. 6 illustrates another example of a method sensing the motion of the user of a wearable device, in accordance with an alternative embodiment.

In operation 610, the method estimates a position of the wearable device using an acceleration sensor embedded within the wearable device. The method estimates the position of the wearable device based on a motion signal sensed from a motion of the user. For example, the method estimates a current position of the wearable device by calculating a distance and a direction travelled from a previous position to the current position of the wearable device, based on an acceleration signal sensed by the acceleration sensor. In this example, the distance travelled and the direction travelled may be calculated from a double integration of the acceleration signal.

In operation 620, the method requests a position of a neighbor wearable device using an NFC unit. The method requests at least two neighbor wearable devices for position related information including a position each of the at least two neighbor wearable devices.

In operation 630, the method updates the position of the neighbor wearable device using a processing unit. The received position of the neighbor wearable device includes a position estimated by the neighbor wearable device through self-motion sensing and a position in which the estimated position is corrected based on the distance travelled.

In operation 640, the method estimates a distance from the neighbor wearable device using the processing unit. The method estimates distances from at least two neighbor wearable devices based on position related information. In this example, the estimated position of the wearable device may be corrected by determining, as the error range, an overlapping space of positions of the wearable device determined based on the estimated distances. A process of determining the error range will be further described with reference to FIG. 7.

In operation 650, the method determines whether the position estimated error range is less than a threshold using the processing unit. In one example, when the error range is provided in a three-dimensional (3D) space, the threshold may be defined based on a volume unit. If the error range is provided in a two-dimensional (2D) space, the threshold may be defined based on an area unit. If the error range is provided in a one-dimensional (1D) space, the threshold may be defined based on a length unit.

When the error range is less than the threshold, in operation 660, the method updates the current position of the wearable device. Using the processing unit, the method determines that the position estimated in operation 610 is the position corrected within the error range less than the threshold of operation 640. The corrected position of the wearable device may be a finally estimated position of the wearable device.

FIG. 7 illustrates an example of a process of a wearable device to estimate a position, in accordance with an embodiment.

Referring to FIG. 7. the wearable device estimates a position 710 of the wearable device through self-motion sensing based on positions 701, 702, and 703 of neighbor wearable devices and distances d1, cm2, and d3 from the neighbor wearable devices. The positions 701, 702, and 703 and the distances d1, d2, and d3 may be estimated using a method as described in FIG. 3 through FIG. 6.

For example, a distance between a wearable device and a first neighbor wearable device is estimated as d1, a distance between the wearable device and a second neighbor wearable device is estimated as d2, and a distance between the wearable device and a third neighbor wearable device is estimated as d3, based on signal attenuation in an NFC channel. Also, the position 710 of the wearable device estimated by the wearable device through self-motion sensing is expressed as an approximate range as illustrated in FIG. 7. The estimated position 710 may be shown through various shapes of ranges based on the motion of the user, without being constrained to a circular shape.

The estimated position 710 is corrected by determining, as the error range, an overlapping space 750 of positions of the wearable device determined based on the distances d1, d2, and d3 from the respective neighbor wearable devices. The corrected position of the wearable device is determined to be an estimated position of the wearable device.

For example, a position of the wearable device is estimated to be present within a radius, for example, the distances d1, d2, and d3, based on each corresponding position 701, 702, and 703 of the neighbor wearable devices. The neighbor wearable devices may be three-dimensionally arranged. Accordingly, the overlapping space 750 of positions estimated based on the distances d1, d2, and d3 from the neighbor wearable devices indicate an overlapping space of spheres using d1, d2, and d3 as a radius based on the positions 701, 702, and 703 of the neighbor wearable devices. When the neighbor wearable devices are two-dimensionally arranged, the overlapping space 750 may refer to an overlapping space of circles.

According to an embodiment, an error may occur in distance estimation due to, for instance, noise in an NFC channel. Accordingly, an overlapping space of positions determined based on distances from neighbor wearable devices are a center position of the positions estimated based on the distances from the neighbor wearable devices. As a result, a more accurate position may be obtained of the wearable device. The overlapping space may be determined to be an error range of a finally estimated position.

According to another embodiment, when a single neighbor wearable device is present, an overlapping space between a position of a wearable device determined based on a distance from the neighbor wearable device and a position of the wearable device determined through self-motion sensing may be determined to be an error range.

The units described herein may be implemented using hardware components. For example, the hardware components may include microphones, amplifiers, band-pass filters, audio to digital convertors, and processing devices. A processing device may be implemented using one or more general-purpose or special purpose computers, such as, for example, a processor, a controller and an arithmetic logic unit, a sensor, a communicator, a digital signal processor, a microcomputer, a field programmable array, a programmable logic unit, a microprocessor or any other device capable of responding to and executing instructions in a defined manner. The processing device may run an operating system (OS) and one or more software applications that run on the OS. The processing device also may access, store, manipulate, process, and create data in response to execution of the software. For purpose of simplicity, the description of a processing device is used as singular; however, one skilled in the art will appreciated that a processing device may include multiple processing elements and multiple types of processing elements. For example, a processing device may include multiple processors or a processor and a controller. In addition, different processing configurations are possible, such a parallel processors.

The method may be performed through software, which may include a computer program, a piece of code, an instruction, or some combination thereof, for independently or collectively instructing or configuring the processing device to operate as desired. Software and data may be embodied permanently or temporarily in any type of machine, component, physical or virtual equipment, a non-transitory computer storage medium or device, or in a propagated signal wave capable of providing instructions or data to or being interpreted by the processing device. The software also may be distributed over network coupled computer systems so that the software is stored and executed in a distributed fashion. In particular, the software and data may be stored by one or more non-transitory computer readable recording mediums. The non-transitory computer readable recording medium may include any data storage device that can store data which can be thereafter read by a computer system or processing device. Examples of the non-transitory computer readable recording medium include read-only memory (ROM), random-access memory (RAM), CD-ROMs, magnetic tapes, floppy disks, optical data storage devices. Also, functional programs, codes, and code segments for accomplishing the example embodiments disclosed herein can be easily construed by programmers skilled in the art to which the embodiments pertain based on and using the flow diagrams and block diagrams of the figures and their corresponding descriptions as provided herein.

The methods according to the above-described embodiments may be recorded, stored, or fixed in one or more non-transitory computer-readable media that includes program instructions to be implemented by a computer to cause a processor to execute or perform the program instructions. The media may also include, alone or in combination with the program instructions, data files, data structures, and the like. The program instructions recorded on the media may be those specially designed and constructed, or they may be of the kind well-known and available to those having skill in the computer software arts. Examples of non-transitory computer-readable media include magnetic media such as hard disks, floppy disks, and magnetic tape; optical media such as CD ROM disks and DVDs; magneto-optical media such as optical discs; and hardware devices that are specially configured to store and perform program instructions, such as read-only memory (ROM), random access memory (RAM), flash memory, and the like. Examples of program instructions include both machine code, such as produced by a compiler, and files containing higher level code that may be executed by the computer using an interpreter. The described hardware devices may be configured to act as one or more software modules in order to perform the operations and methods described above, or vice versa.

A number of examples have been described above. Nevertheless, it should be understood that various modifications may be made. For example, suitable results may be achieved if the described techniques are performed in a different order and/or if components in a described system, architecture, device, or circuit are combined in a different manner and/or replaced or supplemented by other components or their equivalents.

## Claims

1. A wearable device (100, 200), comprising:
a motion sensor (210) configured to sense a motion signal from a motion of a user of the wearable device (100, 200); and
a processor (230) configured to calculate a motion of the wearable device (200) based on the motion signal and position related information received from a neighbor wearable device (101, 102, 103); and
a magnetic near field communicator (220) configured to receive the position related information from the neighbor wearable device and transmit the position related information to the processor,
wherein the position related information comprises at least one of a distance from the wearable device estimated by the neighbor wearable device, a motion signal of the neighbor wearable device, and a position of the neighbor wearable device.

2. The wearable device of claim 1, wherein the processor is further configured to estimate a position (710) of the wearable device based on the motion signal,
preferably, wherein the processor is further configured to correct the estimated position (710) of the wearable device based on the position related information from neighbor wearable devices whose number varies depending on a degree of accuracy needed so that an error range (750) of the estimated position is less than a threshold.

3. The wearable device of claim 2, wherein the error range (750) is an overlapping space of positions of the wearable device estimated based on distances from at least two neighbor wearable devices calculated from the position related information.

4. The wearable device of one of the preceding claims, wherein the processor (230) is further configured to estimate a distance from the neighbor wearable device (101, 102, 103) based on a power level of a reference signal included in the position related information, and to calculate a position of the wearable device based on the estimated distance and the position related information, and/or
wherein the processor is further configured to sense the motion of the user by tracking a position of the wearable device, in time series, based on the motion signal and the position related information.

5. The wearable device of one of the preceding claims,
wherein the motion sensor comprises at least one of an acceleration sensor and an electromyogram (EMG) sensor.

6. The wearable device of one of the preceding claims, further comprising:
a body applier configured to apply the wearable device to a body of the user.

7. A method of a wearable device, the method comprising:
Sensing (510) a motion signal from a motion of a user of the wearable device;
receiving (520) position related information from a first neighbor wearable device using a magnetic near field communication unit; and
calculating (530) a motion of the wearable device based on the motion signal and position related information received from the first neighbor wearable device wherein the position related information comprises at least one of a distance from the wearable device estimated by the neighbor wearable device, a motion signal of the neighbor wearable device, and a position of the neighbor wearable device.

8. The method of claim 7, wherein the sensing comprises estimating a position of the wearable device based on the motion signal,
preferably, wherein the calculating comprises correcting the estimated position of the wearable device based on the position related information from neighbor wearable devices whose number varies depending on a degree of accuracy needed so that an error range of the estimated position is less than a threshold,
more preferably, wherein the correcting comprises:
detecting a power level of a reference signal included in the position related information;
estimating a distance of the wearable device from the first neighbor wearable device based on the detected power level; and
determining the error range based on the motion signal and the distance from the first neighbor wearable device.

9. The method of one of claims 7 or 8, wherein the calculating comprises:
estimating a distance of the wearable device from the first neighbor wearable device by analyzing a power level of a reference signal included in the position related information; and
calculating a position of the wearable device based on the estimated distance from the first neighbor wearable device and a position of the first neighbor wearable device that is included in the position related information.

10. The method of one of claims 7 - 9, the method comprising:
Estimating a position of the wearable device based on the sensed motion signal; and
wherein calculating a motion of the wearable device is based on the estimated position of the wearable device and position related information from at least two neighbor wearable devices including the first neighbor wearable device,
preferably, wherein the calculating comprises:
estimating distances from the at least two neighbor wearable devices based on the position related information; and
correcting the estimated position of the wearable device by determining, as an error range, an overlapping space of positions of the wearable device based on the distances from the at least two neighbor wearable devices,
more preferably, wherein the calculating comprises:
determining the corrected position of the wearable device as a finally estimated position of the wearable device when the error range is less than a threshold.

11. The method of a wearable device of claim 10, further comprising the step of:
correcting the estimated position of the wearable device to have an error range less than a threshold by determining an overlapping space of positions of the wearable device determined based on distances from the neighbor wearable devices.

12. The method of claim 11, wherein the error range is an overlapping space of positions of the wearable device estimated based on distances from the at least two neighbor wearable devices calculated from the position related information.

13. The method of claim 11 or 12, further comprising:
estimating a distance from the at least two neighbor wearable devices based on a power level of a reference signal included in the position related information, and calculating a position of the wearable device based on the estimated distance and the position related information, and/or
updating the position of the wearable device as a finally estimated position of the wearable device in response to the error range being less than the threshold.

14. The method of one of claims claim 11 - 13, wherein the estimating of the position of the wearable device comprises
calculating a distance and a direction travelled from a previous position to a current position of the wearable device based on a double integration of an acceleration signal.

15. A non-transitory computer-readable medium comprising at least one program for instructing a computer to perform the method of claim 7.

## Patentansprüche

1. Tragbares Gerät (100, 200), das umfasst:
einen Bewegungssensor (210), der konfiguriert ist zum Erfassen eines Bewegungssignals von einer Bewegung eines Benutzers des tragbaren Geräts (100, 200),
einen Prozessor (230), der konfiguriert ist zum Berechnen einer Bewegung des tragbaren Geräts (200) basierend auf dem Bewegungssignal und auf positionsbezogenen Informationen, die von einem benachbarten tragbaren Gerät (101, 102, 103) empfangen werden, und
eine Magnetisches-Nahfeld-Kommunikationseinheit (220), die konfiguriert ist zum Empfangen von positionsbezogenen Informationen von dem benachbarten tragbaren Gerät und zum Senden der positionsbezogenen Informationen an den Prozessor,
wobei die positionsbezogenen Informationen eine Distanz von dem tragbaren Gerät, die durch das benachbarte tragbare Gerät geschätzt wird, ein Bewegungssignal des benachbarten tragbaren Geräts und/oder eine Position des benachbarten tragbaren Geräts umfassen.

2. Tragbares Gerät nach Anspruch 1, wobei der Prozessor weiterhin konfiguriert ist zum Schätzen einer Position (710) des tragbaren Geräts basierend auf dem Bewegungssignal,
wobei der Prozessor vorzugsweise weiterhin konfiguriert ist zum Korrigieren der geschätzten Position (710) des tragbaren Geräts basierend auf den positionsbezogenen Informationen von benachbarten tragbaren Geräten, deren Anzahl in Abhängigkeit von dem Genauigkeitsgrad abhängt, der erforderlich ist, damit ein Fehlerbereich (750) der geschätzten Position kleiner als ein Schwellwert ist.

3. Tragbares Gerät nach Anspruch 2, wobei der Fehlerbereich (750) ein überlappender Raum von Positionen des tragbaren Geräts ist, die basierend auf Distanzen von wenigstens zwei benachbarten tragbaren Geräten, die aus den positionsbezogenen Informationen berechnet werden, geschätzt werden.

4. Tragbares Gerät nach einem der vorstehenden Ansprüche, wobei der Prozessor (230) weiterhin konfiguriert ist zum Schätzen der Distanz von dem benachbarten tragbaren Gerät (101, 102, 103) basierend auf dem Leistungspegel eines in den positionsbezogenen Informationen enthaltenen Referenzsignals und zum Berechnen der Position des tragbaren Geräts basierend auf der geschätzten Distanz und den positionsbezogenen Informationen, - und/oder
wobei der Prozessor weiterhin konfiguriert ist zum Erfassen der Bewegung des Benutzers durch das Verfolgen der Position des tragbaren Geräts in einer Zeitreihe basierend auf dem Bewegungssignal und den positionsbezogenen Informationen.

5. Tragbares Gerät nach einem der vorstehenden Ansprüche,
wobei der Bewegungssensor einen Beschleunigungssensor und/oder einen Elektromyogramm (EMG)-Sensor umfasst.

6. Tragbares Gerät nach einem der vorstehenden Ansprüche, das weiterhin umfasst:
eine Körperanbringungseinrichtung, die konfiguriert ist zum Anbringen des tragbaren Geräts am Körper des Benutzers.

7. Verfahren für ein tragbares Gerät, wobei das Verfahren umfasst:
Erfassen (510) eines Bewegungssignals von einer Bewegung eines Benutzers des tragbaren Geräts,
Empfangen (520) von positionsbezogenen Informationen von einem ersten benachbarten tragbaren Gerät unter Verwendung einer Magnetisches-Nahfeld-Kommunikationseinheit, und
Berechnen (530) einer Bewegung des tragbaren Geräts basierend auf dem Bewegungssignal und auf von dem ersten benachbarten tragbaren Gerät empfangenen positionsbezogenen Informationen,
wobei die positionsbezogenen Informationen eine Distanz von dem tragbaren Gerät, die durch das benachbarte tragbare Gerät geschätzt wird, ein Bewegungssignal des benachbarten tragbaren Geräts und/oder eine Position des benachbarten tragbaren Geräts umfassen.

8. Verfahren nach Anspruch 7, wobei das Erfassen das Schätzen einer Position des tragbaren Geräts basierend auf dem Bewegungssignal umfasst,
wobei das Berechnen vorzugsweise das Korrigieren der geschätzten Position des tragbaren Geräts basierend auf den positionsbezogenen Informationen von benachbarten tragbaren Geräten, deren Anzahl in Abhängigkeit von dem Genauigkeitsgrad abhängt, der erforderlich ist, damit ein Fehlerbereich der geschätzten Position kleiner als ein Schwellwert ist,
und wobei das Korrigieren vorzugsweise umfasst:
Erfassen eines Leistungspegels eines in den positionsbezogenen Informationen enthaltenen Referenzsignals,
Schätzen der Distanz des tragbaren Geräts von dem ersten benachbarten tragbaren Gerät basierend auf dem erfassten Leistungspegel, und
Bestimmen des Fehlerbereichs basierend auf dem Bewegungssignal und der Distanz von dem ersten benachbarten tragbaren Gerät.

9. Verfahren nach einem der Ansprüche 7 oder 8, wobei das Berechnen umfasst:
Schätzen der Distanz des tragbaren Geräts von dem ersten benachbarten tragbaren Gerät durch das Analysieren des Leistungspegels eines in den positionsbezogenen Informationen enthaltenen Referenzsignals, und
Berechnen der Position des tragbaren Geräts basierend auf der geschätzten Distanz von dem ersten benachbarten tragbaren Gerät und auf der in den positionsbezogenen Informationen enthaltenen Position des ersten benachbarten tragbaren Geräts.

10. Verfahren nach einem der Ansprüche 7-9, wobei das Verfahren umfasst:
Schätzen der Position des tragbaren Geräts basierend auf dem erfassten Bewegungssignal,
wobei das Berechnen einer Bewegung des tragbaren Geräts auf der geschätzten Position des tragbaren Geräts und auf positionsbezogenen Informationen von wenigstens zwei benachbarten tragbaren Geräten einschließlich des ersten benachbarten tragbaren Geräts basiert,
wobei das Berechnen vorzugsweise umfasst:
Schätzen der Distanzen von den wenigstens zwei benachbarten tragbaren Geräten basierend auf den positionsbezogenen Informationen, und
Korrigieren der geschätzten Position des tragbaren Geräts durch das Bestimmen, als eines Fehlerbereichs, eines überlappenden Raums von Positionen des tragbaren Geräts basierend auf den Distanzen von den wenigstens zwei benachbarten tragbaren Geräten,
wobei das Berechnen vorzugsweise umfasst:
Bestimmen der korrigierten Position des tragbaren Geräts als einer endgültig geschätzten Position des tragbaren Geräts, wenn der Fehlerbereich kleiner als ein Schwellwert ist.

11. Verfahren für ein tragbares Gerät nach Anspruch 10, das weiterhin den folgenden Schritt umfasst:
Korrigieren der geschätzten Position des tragbaren Geräts mit einem Fehlerbereich, der kleiner als ein Schwellwert ist, durch das Bestimmen eines überlappenden Raums von Positionen des tragbaren Geräts, die basierend auf Distanzen von den benachbarten tragbaren Geräten bestimmt werden.

12. Verfahren nach Anspruch 11, wobei der Fehlerbereich ein überlappender Raum von Positionen des tragbaren Geräts ist, die basierend auf Distanzen von den wenigstens zwei benachbarten tragbaren Geräten, die aus den positionsbezogenen Informationen berechnet werden, geschätzt werden.

13. Verfahren nach Anspruch 11 oder 12, das weiterhin umfasst:
Schätzen der Distanz von den wenigstens zwei benachbarten tragbaren Geräten basierend auf dem Leistungspegel eines in den positionsbezogenen Informationen enthaltenen Referenzsignals, und Berechnen der Position des tragbaren Geräts basierend auf der geschätzten Distanz und den positionsbezogenen Informationen, und/oder
Aktualisieren der Position des tragbaren Geräts als einer endgültig geschätzten Position des tragbaren Geräts in Reaktion darauf, dass der Fehlerbereich kleiner als der Schwellwert ist.

14. Verfahren nach einem der Ansprüche 11-13, wobei das Schätzen der Position des tragbaren Geräts umfasst:
Berechnen der Distanz und der Richtung von einer vorausgehenden Position zu einer aktuellen Position des tragbaren Geräts basierend auf einer doppelten Integration eines Beschleunigungssignals.

15. Nicht-transitorisches, computerlesbares Medium, das wenigstens ein Programm für das Anweisen eines Computers zum Durchführen des Verfahrens von Anspruch 7 umfasst.

## Revendications

1. Dispositif portable (100, 200) comprenant :
un capteur de mouvement (210) configuré pour détecter un signal de mouvement généré par le mouvement d'un utilisateur du dispositif portable (100, 200), et
un processeur (230) configuré pour calculer un mouvement du dispositif portable (200) sur la base du signal de mouvement et d'informations liées à la position reçues d'un dispositif portable (101, 102, 103) voisin, et
un communicateur en champ proche magnétique (220) configuré pour recevoir les informations liées à la position sur le dispositif portable voisin et pour transmettre au processeur les informations liées à la position,
dans lequel les informations liées à la position comprennent au moins l'un d'une distance au dispositif portable, estimée par le dispositif portable voisin, d'un signal de mouvement du dispositif portable voisin et d'une position du dispositif portable voisin.

2. Dispositif portable selon la revendication 1, dans lequel le processeur est en outre configuré pour estimer la position (710) du dispositif portable sur la base du signal de mouvement,
dans lequel, de préférence, le processeur est configuré de plus pour corriger la position estimée (710) du dispositif portable sur la base des informations liées à la position par rapport aux dispositifs portables voisins dont le nombre varie en fonction du degré de précision nécessaire pour que la plage d'erreur (750) de la position estimée soit inférieure à un seuil.

3. Dispositif portable selon la revendication 2, dans lequel la plage d'erreur (750) est un espace de chevauchement de positions du dispositif portable qui est estimé sur la base de distances à au moins deux dispositifs portables voisins, calculées à partir les informations liées à la position.

4. Dispositif portable selon l'une des revendications précédentes, dans lequel le processeur (130) est en outre configuré pour estimer une distance au dispositif portable voisin (101, 102, 103) sur la base du niveau de puissance d'un signal de référence inclus dans les informations liées à la position, ainsi que pour calculer la position du dispositif portable sur la base de la distance estimée et des informations liées à la position, et/ou
dans lequel le processeur est en outre configuré pour détecter le mouvement de l'utilisateur en suivant une position du dispositif portable, en série dans le temps, sur la base du signal de mouvement et des informations liées à la position.

5. Dispositif portable selon l'une des revendications précédentes,
dans lequel le capteur de mouvement comprend au moins l'un d'un capteur d'accélération et d'un capteur d'électromyogramme (EMG).

6. Dispositif portable selon l'une des revendications précédentes, comprenant en outre :
un dispositif d'application au corps configuré pour appliquer le dispositif portable sur le corps de l'utilisateur.

7. Procédé d'un dispositif portable, le procédé comprenant :
la détection (510) d'un signal de mouvement généré par le mouvement d'un utilisateur du dispositif portable,
la réception (520) d'informations liées à la position à un premier dispositif portable voisin en utilisant une unité de communication en champ proche magnétique, et
le calcul (530) d'un mouvement du dispositif portable sur la base du signal de mouvement et d'informations liées à la position reçues depuis le premier dispositif portable voisin,
dans lequel les informations liées à la position comprennent au moins l'un d'une distance au dispositif portable, estimée par le dispositif portable voisin, du signal de mouvement du dispositif portable voisin et de la position du dispositif portable voisin.

8. Procédé selon la revendication 7, dans lequel la détection comprend l'estimation de la position du dispositif portable sur la base du signal de mouvement,
de préférence, le calcul comprenant la correction de la position estimée du dispositif portable sur la base des informations liées à la position à des dispositifs portables voisins dont le nombre varie en fonction du degré de précision nécessaire pour que la plage d'erreur de la position estimée soit inférieure à un seuil,
encore mieux, dans lequel la correction comprend :
la détection du niveau de puissance d'un signal de référence inclus dans les informations liées à la position,
l'estimation de la distance du dispositif portable au premier dispositif portable voisin sur la base du niveau de puissance détecté, et
la détermination de la plage d'erreur sur la base du signal de mouvement et de la distance au premier dispositif portable voisin.

9. Procédé selon l'une des revendications 7 ou 8, dans lequel le calcul comprend :
l'estimation de la distance du dispositif portable au premier dispositif portable voisin en analysant un niveau de puissance d'un signal de référence inclus dans les informations liées à la position, et
le calcul de la position du dispositif portable sur la base de la distance estimée au premier dispositif portable voisin et de la position du premier dispositif portable voisin qui est incluse dans les informations liées à la position.

10. Procédé selon l'une des revendications 7 à 9, le procédé comprenant :
l'estimation de la position du dispositif portable sur la base du signal de mouvement détecté, et
dans lequel le calcul d'un mouvement du dispositif portable est fondé sur la position estimée du dispositif portable et sur les informations liées à la position à au moins deux dispositifs portables voisins y compris le premier dispositif portable voisin,
de préférence, le calcul comprenant :
l'estimation des distances aux deux dispositifs portables voisins ou plus sur la base des informations liées à la position, et
la correction de la position estimée du dispositif portable en déterminant, comme plage d'erreur, un espace de chevauchement de positions du dispositif portable sur la base des distances par rapport aux deux dispositifs portables voisins ou plus,
idéalement, le calcul comprenant :
la détermination de la position corrigée du dispositif portable comme position estimée finalement du dispositif portable lorsque la plage d'erreur est inférieure à un seuil.

11. Procédé d'un dispositif portable selon la revendication 10, comprenant en outre l'étape suivante :
la correction de la position estimée du dispositif portable pour obtenir une plage d'erreur inférieure à un seuil en déterminant un espace de chevauchement des positions du dispositif portable défini sur la base de distances aux dispositifs portables voisins.

12. Procédé selon la revendication 11, dans lequel la plage d'erreur est un espace de chevauchement de positions du dispositif portable estimé sur la base de distances aux deux dispositifs portables voisins ou plus calculées à partir des informations liées à la position.

13. Procédé selon la revendication 11 ou la revendication 12, comprenant en outre :
l'estimation de la distance aux deux dispositifs portables voisins ou plus sur la base d'un niveau de puissance d'un signal de référence inclus dans les informations liées à la position, ainsi que le calcul de la position du dispositif portable sur la base de la distance estimée et des informations liées à la position, et/ou
la mise à jour de la position du dispositif portable en tant que position estimée finalement du dispositif portable en réponse à la plage d'erreur en dessous du seuil.

14. Procédé selon l'une des revendications 11 à 13, dans lequel l'estimation de la position du dispositif portable comprend :
le calcul d'une distance et d'une direction parcourue depuis une position précédente jusqu'à la position actuelle du dispositif portable sur la base d'une double intégration d'un signal d'accélération.

15. Support non transitoire pouvant être lu par ordinateur comprenant au moins un programme destiné à ordonner à un ordinateur d'exécuter le procédé de la revendication 7.
